# EUROPEAN PATENT SPECIFICATION

(11) **EP 3 818 362 B1**
(45) Date of publication and mention of the grant of the patent: **07.06.2023**
(21) Application number: 19737075.2
(22) Date of filing: 05.07.2019
(51) Int. Cl.: G01N 27/12, G01N 33/00, G01N 27/02

(54) **GAS SENSOR**
GASSENSOR
CAPTEUR DE GAZ

(30) Priority: 06.07.2018 GB 201811114
(43) Date of publication of application: 12.05.2021
(73) Proprietor: B-SENS, 7000 Mons (BE)
(72) Inventor: DEBLIQUY, Marc, 7850 Petit-Enghien (BE); LAHEM, Driss, 7340 Colfontaine (BE); CAUCHETEUR, Christophe, 7000 Mons (BE)
(74) Representative: ABYOO
(86) International application number: PCT/EP2019/068065
(87) International publication number: WO 2020/008020

(56) References cited:
- WO-A1-2018/105975
- US-A1- 2017 350 817
- CH Y WANG ET AL: "Photon stimulated sensor based on indium oxide nanoparticles I: Wide-concentration-range ozone monitoring in air", SENSORS AND ACTUATORS B: CHEMICAL, ELSEVIER BV, NL, vol. 152, no. 2, 8 December 2010 (2010-12-08), pages 235-240, XP028169937, ISSN: 0925-4005, DOI: 10.1016/J.SNB.2010.12.014 [retrieved on 2010-12-16]
- Z. Q. ZHENG ET AL: "Light-controlling, flexible and transparent ethanol gas sensor based on ZnO nanoparticles for wearable devices", SCIENTIFIC REPORTS, vol. 5, no. 1, 16 June 2015 (2015-06-16), XP055617841, DOI: 10.1038/srep11070
- CHAO ZHANG ET AL: "Room temperature responses of visible-light illuminated WO3 sensors to NO2 in sub-ppm range", SENSORS AND ACTUATORS B: CHEMICAL, vol. 181, 7 February 2013 (2013-02-07), pages 395-401, XP055617515, NL ISSN: 0925-4005, DOI: 10.1016/j.snb.2013.01.082
- Yi Chen ET AL: "UV activated hollow ZnO microspheres for selective ethanol sensors at low temperatures", Sensors and Actuators B: Chemical, vol. 232, 1 September 2016 (2016-09-01), pages 158-164, XP055733380, NL ISSN: 0925-4005, DOI: 10.1016/j.snb.2016.03.138
- JOSHI NIRAV ET AL: "One-step approach for preparing ozone gas sensors based on hierarchical NiCo2O4 structures", RSC ADVANCES, vol. 6, no. 95, 1 January 2016 (2016-01-01), pages 92655-92662, XP055947511, DOI: 10.1039/C6RA18384K Retrieved from the Internet: URL:https://pubs.rsc.org/en/content/articl epdf/2016/ra/c6ra18384k>

## Description

The present invention relates to a gas sensor for detecting and measuring the presence and/or quantity of one of more gasses, notably in ambient air.

Whilst known gas sensors, for example, chemoresistor or semiconductor gas sensors, can work well in specific circumstances for which they have been designed, they do not always provide a combination of features for all applications. For example, such sensors require a heat source, generally an electrically powered heating circuit, to confer acceptable response and recovery times; this increases power consumption, presents an explosion or fire risk if used in a flammable or explosive atmosphere, contributes to complexity and cost of the sensors and requires the use of temperature resistant substrates such as ceramics.

There is thus a need for an improved gas detector, notably a gas sensor which is easier to handle, has fast recovery and is adapted to work at room temperature, notably in ambient air.

Gas sensors are known from the articles

*"*Photon stimulated sensor based on indium oxide nanoparticles I: Wide-concentration-range ozone monitoring in air", Ch. Y. Wang et al, Sensors and Actuators B: Chemical, vol. 152, no. 2, 8 December 2010, pages 235-240,

*"*Light-controlling, flexible and transparent ethanol gas sensor based on ZnO nanoparticles for wearable devices", Z. Q. Zheng et al, Scientific Reports, vol. 5, no. 1, 16 June 2015,

*"*UV activated hollow ZnO microspheres for selective ethanol sensors at low temperatures", Y. Chen et al, Sensors and Actuators B: Chemical, vol. 232, 1 September 2016, pages 158-164, and
from the patent publication WO2018105975 A1.

According to one of its aspects, the present invention provides a gas sensor as defined in independent claim 1. A method of detecting a target gas in a gaseous atmosphere is defined in independent method claim 15. The dependent claims 2 to 14 define preferred or alternative embodiments.

As used herein, the term "electrical impedance" means the total opposition that a circuit or a part of a circuit presents to electric current. The electrical impedance (also known as complex impedance) comprises the resistance (the real part of the impedance) and the reactance (the imaginary part of the impedance).

The transparent substrate of the gas sensor may be flexible; it may be rigid. Use of a flexible substrate facilitates application of the substrate to a wide variety of supports, irrespective of the shape of the support. Use of a flexible substrate also facilitates compatibility with manufacturing techniques used, for example, to deposit electrodes, for example by printing, screen printing or photolithography, and/or to separate individual sensor components, for example by cutting, from a single transparent sheet upon which a plurality of sensor components are manufactured.

The transparent substrate may be made of a single film or comprise a plurality of overlaid films. Particularly in the case of a flexible transparent substrate, the transparent substrate may comprise a plastics film, notably a plastics film selected from PET, PE, PEN, PTFE, PVDF, a polymer, a non-crystalline polymer, a bio-sourced plastics such as PLA, a polylactide, and combination thereof. In the case of a rigid transparent substrate, the transparent substrate may comprise glass.

The target gas to be detected may be or comprise: NO₂ gas; Os gas; H₂ gas; CO gas; VOC's (Volatile Organic Compound); combination thereof. Such detection may be useful for monitoring atmospheric pollution in air and/or the concentration of the target gas in a gaseous atmosphere.

The gaseous atmosphere may comprise or consist essentially of air. The gas sensor may be used in ambient air, for example inside a building, outside a building or in an open air space, notably to measure or monitor air pollutant gases. It may be used, for example, in road tunnels, car parks, storage halls, floor voids, cable ducts or sewers. It may be used for gas leak detection or for detecting or monitoring of the target gas in a large open space, for example a park or a city street. Where the gas sensor is used for air pollution and/or gas detection this provides an easily installed and cost efficient system for large areas. The gaseous atmosphere in which the target gas is detected may be exhaust gas orflue gas for example from a domestic or industrial chimney or from an exhaust system from an engine, for example a car engine. The gas sensor may be used for monitoring NO₂ gas and/or ozone gas in ambient air.

The gas sensitive detection material may comprise, consist or consist essentially of a metal oxide and/or graphene. The gas sensitive detection material may be combined with a gas capturing material, the gas capturing material being configured to facilitate capturing of gas to be detected by the gas sensitive detection material. The gas capturing material may comprise a conductive polymer and/or an organic material, for example an organic material selected from porphyrins, phthalocyanines, organometallic complexes and combination thereof. The metal oxide may be a doped metal oxide and/or a non-stoichiometric metal oxide, notably a sub-stoichiometric metal oxide. The metal of the metal oxide may be or comprise: Fe; La; Cr; Cu; In; Zn; Sn; W; Ni; combination thereof. The graphene may be a graphene oxide.

As used herein, the term "consist essentially of" is intended to limit a definition or the scope of a claim to the specified materials or steps and those that do not materially affect the basic and novel characteristic(s) of the claimed invention.

The gas sensitive detection material is preferably porous; according to the invention, it is in the form of nanoplatelets. A particular form provides a better sensitivity to the gas sensor due to higher surface adsorption sites and higher contact surface area available between the target gas and the gas sensitive detection material. The nanoplatelets may have an average thickness of ≤ 2 µm; ≤ 1µm; ≤ 500 nm; ≤ 300 nm or ≤ 200 nm. Preferably, the nanoplatelets have a thickness in the range from 50 to 100 nm. According to the invention; the nanoplatelets are arranged in the form of hollow spheres or hierarchical hollow spheres, notably hollow spheres having an external mean diameter of ≤ 50µm, preferably, ≤ 20 µm, more preferably ≤ 10µm. Hierarchical structures prevent the aggregation of nanomaterials and greatly increase the specific surface area. The hollow spheres form provides permeability and further increases the sensitivity of the gas sensor by i) increasing the scattering of light within the gas sensitive detective material and ii) increasing the gas diffusion rate and pathways. Use of a porous gas sensitive detection material, particularly in the form of particles or nanoplatelets and more particularly in the form or hollow spheres facilitates configuration of a gas sensor adapted for operation at room temperature, notably at 20°C.

The gas sensitive detection material may have a thickness of ≥ 50 nm and/or ≤ 50 µm, preferably in the range 4-20 µm.

The light source is intended to provide activation energy to the gas sensitive detection layer. Preferably the light source provides the only source of activation energy (other than any heat derived from the surroundings of the sensor); thus, it is preferred that the sensor and/or its surroundings are not provided with a dedicated heat source, for example a heating coil or heating resistance, for heating or activating the gas sensitive detection layer; this simplifies configuration of the sensor and avoids the need for providing energy for such heating. The light source which is configured to provide light to the gas sensitive detection layer through the transparent substrate may comprise a light-emitting diode. Light emitting diodes (LED) are particularly adapted for such gas sensor as they provide light in an energy efficient manner, and provide a light beam of a size compatible with the size of the gas sensitive detection layer and which may be easily focused. Whilst a LED may produce some heat energy in addition to light, the heating effect of the LED, or indeed any other light source, upon the gas sensitive detection layer is preferably small or negligible. The light source may be a white light source or a monochromatic light source. As used herein, the term "monochromatic light source" means a light source emitting a relatively narrow band of wavelengths which is perceived by the naked eye as a single colour. As used herein, the term "white light source" means a light source comprising at least a plurality of wavelengths which appears substantially white to the naked eye. Preferably the light source is a monochromatic light source, for example a blue light source, notably a blue light source configured to emit wavelengths in the range from 460 to 490 nm. The wavelengths of the monochromatic, for example blue, light source is preferably selected to correspond to wavelengths which are effective for activating the gas sensitive detection layer; this avoids expending energy in producing light at wavelengths which are less effective or ineffective for activating the gas sensitive detection layer. The light source may have a light intensity of at least 10 mcd, preferably at least 100 mcd, more preferably at least 500 mcd and/or a light intensity of less than 5 cd, preferably less than 2 cd.

The gas sensor may comprise a gas filter, notably between the gas sensitive detection material and the gaseous atmosphere. Preferably, the gas filter is configured to prevent or reduce the concentration of one or more interfering gases from the gaseous atmosphere from contacting the gas sensitive detection layer. The interfering gas from the gaseous atmosphere may be or comprise: NO₂ gas; Os gas; H₂ gas; CO gas; VOC's; combination thereof. Such a gas filter may be useful for avoiding interference from an interfering gas when monitoring a particular target gas and if the gas sensitive detection material is sensitive to a plurality of gases present in the gaseous atmosphere. The interfering gases may depend on the intended applications and context and may be the same as the previously mentioned target gases. For example, ozone may be an interfering gas for the detection of NO₂ and NO₂ may be considered as an interfering gas if the goal is to measure ozone in air. The gas sensor may comprise a mechanical packaging which may surround and/or block and/or be applied on or around the gas sensitive detection material, for example a metallic grid or a sintering, notably a ceramic sintering. The packaging may comprise a filter. For example, the packaging may comprise a sintered material having a filtering material or a metallic grid retaining a filter.

The gas sensor may detect concentration of target gas of ≥1 ppb or ≥5ppb and/or ≥ 20ppb or ≥100ppb and/or ≤1 ppm or ≤10ppm or ≤ 20ppm or ≤ 50ppm or ≤ 300ppm. Particularly when the gas sensor is to be used in safety application, for example the monitoring of CO gas, the gas sensor may detect concentration of target gas in the range 20-200 ppm. Particularly when monitoring ozone, the gas sensor may detect a concentration of Os of ≤1 ppm

Advantageously, the gas sensor detects, in response to a concentration of target gas in the gaseous atmosphere which is less than 1 ppm, preferably less than 500ppb, more preferably less than 100ppb, a variation in electrical impedance of the gas sensitive detection layer which is at least 500 times, preferably at least 5000 times, more preferably at least 10000 times the electrical impedance of the gas sensitive detection layer prior to exposure to the gaseous atmosphere comprising the target gas. The gas sensor may be adapted to detect an electrical impedance of the gas sensitive detection layer, notably at 20°C, in the range from 1kΩ to 100MΩ, particularly when exposed to a concentration of target gas in the gaseous atmosphere which is less than 1 ppm.

The gas sensor may be used for qualitative and/or quantitative measurements. It may detect the absolute amount or concentration of the target gas in the gaseous atmosphere and/or detect the relative amount or change in the amount or concentration of the target gas.

The gas sensor, notably its gas sensitive detection layer, may be adapted to operate at room temperature, notably at 20°C, and/or at outdoor temperatures, for example at temperatures of -20°C, -5°C, 0°C, 5°C, 10°C or 15°C, notably without an additional heat source. The ability to operate at such temperatures without requiring heating of the gas sensitive detection layer avoid problems associated with prior sensors which require heating in order to operate, such as high power consumption and thermally induced grain growth.

Preferably, the gas sensor is substantially insensitive to humidity. It is preferably humidity neutral, that is to say that the difference between the electrical impedance between:
a) a condition wherein the humidity is 20% at a temperature of 20°C and at a pressure of 1 atmosphere; and
b) a condition wherein the humidity is 80% at a temperature of 20°C and at a pressure of 1 atmosphere; and
in which the gas sensor has been exposed to the same quantity of target gas, notably 1ppm in air and/or 0.5ppm in air is less than 50%, preferably less than 20%, more preferably less than 10%, notably after a period of at least 8 hours, preferably a period of at least 4 hours, at least 2 hours, at least 1 hour or at least 30 minutes, with or without external application of energy from an external source.

Preferably, the reversibility of the sensor is such that the difference between the electrical impedance of the gas sensitive detection layer between:
a) a condition prior to being exposed to the target gas, and
b) a condition in which it has been exposed to the target gas and is subsequently exposed to an atmosphere that does not include the target gas;
is less than 20%, preferably less than 10%, more preferably less than 5%, notably after a period of less than 4 hours, preferably a period of less than 2 hours, less than 1 hour or less than 30 minutes, with or without external application of energy from an external source, preferably at ambient atmospheric conditions and notably at a 20°C and 1 atmosphere in ambient or test air.

The connections configured to allow for detection of electrical impedance of the gas sensitive detection material may comprise a pair of spaced electrodes, preferably interdigitated electrodes, each of which is electrically connected to the gas sensitive detection material. Preferably, the connections are supported by the transparent substrate and overlaid by the gas sensitive detection material. In this configuration, the electrodes are in direct proximity to the portion of the gas sensitive detection material into which light from the light source is first received and which thus received the most activation energy from the light source. The distance through the gas sensitive detection material between the electrodes may be ≥ 1 µm and/or ≤ 300 µm.

The gas sensor may be manufactured by:
- Providing electrodes at a surface of the transparent surface;
- Depositing a gas sensitive detection material over the electrodes;
- Providing a light source at the other surface of a transparent substrate so that the light from the light source is directed through the transparent substrate towards the gas sensitive detection material.

According to another aspect, the present invention provides a method of detecting a target gas in a gaseous atmosphere, notably NO₂ or Os gas in air, comprising:
- arranging a gas sensor in the gaseous atmosphere, the gas sensor comprising a) a transparent substrate, b) a gas sensitive detection layer supported by the transparent substrate, the gas sensitive detection layer comprising i) a gas sensitive detection material having an electrical impedance which is sensitive to the target gas and ii) connections configured to allow for detection of electrical impedance of the gas sensitive detection material; and c) a light source configured to provide light to the gas sensitive detection layer through the transparent substrate;
- providing light to the gas sensitive detection layer through the transparent substrate from the light source;
- detecting the presence and/or the concentration of the target gas in the gaseous atmosphere by monitoring the electrical impedance of the gas sensitive detection layer.

An embodiment of the invention will now be described, by way of example only with reference to the accompanying drawings of which:
Fig. 1 is a schematic cross-section (not to scale) of a gas sensor; and
Fig. 2 to 4 are graphs showing responses of the gas sensor.

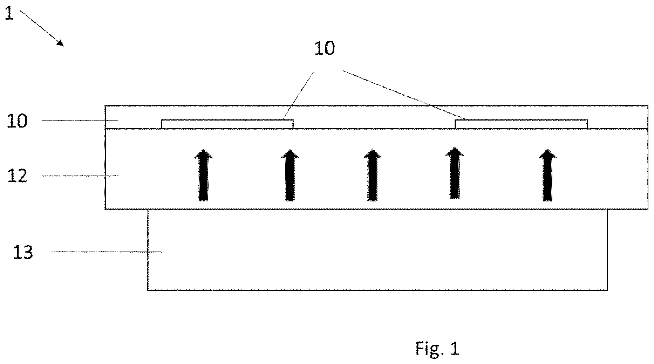

The gas sensor 1 shown in Fig.1 comprises a flexible transparent PET film 12 having a thickness of about 100 µm having on one of its surface printed gold electrodes 11 having a thickness of about 200nm and a gas sensitive detection material 10 comprising hollow spheres of WOs having an external mean diameter of about 2µm. This thickness of the gas sensitive detection material 10 is about 20 µm. On the opposite surface of the PET film, a LED 13 is arranged to provide light towards the gas sensitive detection material 10 and electrodes 11 through the PET film 12.

The gas sensor 1 is manufactured by
- Printing a negative image of the electrodes on the PET film 12 with a laser printer (HP colour laser Jet CP1515) to provide a printed substrate;
- Depositing a gold layer of about 200 nm on the printed substrate by sputtering (Leica EM SCD 500, 10-2 mbar air);
- Revealing the golden electrodes 11 by a lift off in an acetone bath which removes the ink of the negative drawing;
- Screen printing the WOs powder 10 on the front side of the substrate 12;
- Installing a LED lamp 13 on the back side of the substrate 12.

The tungsten oxide (hereinafter written as WOs) powder of the gas sensitive detection material 10 was prepared according to the following process: about 2 mmol Pb(Ac)₂ and about 2 mmol Na₂WO₄ (2 mmol) were dissolved in about 25mL of distilled water, respectively, and then the two solutions were mixed under vigorous magnetic stirring at room temperature. Precipitates were formed quickly, and after that the mixture was transferred into a Teflon-lined stainless steel autoclave at about 160°C for about 5h. After cooling to room temperature, the product was filtered, washed several times with distilled water, and then dried in air at about 70°C. Subsequently, the product was immersed in about 4M HNOs solution for about 48 h to transform PbWO₄ to WO₃.H₂O. Then the products were filtered, washed with distilled water, and dried in air. Next, the acid-treated products were calcined in a furnace at about 500°C for about 2 h in air to obtain the WOs powder. Afterwards, about 3 g of as-synthesized WOs powder was dissolved in the 2.5 mL terpineol solution to form a homogenous paste. Subsequently, the obtained paste was screen-printed on the sensor substrate and the printed sensor was calcined in a furnace at about 400°C to remove terpineol.

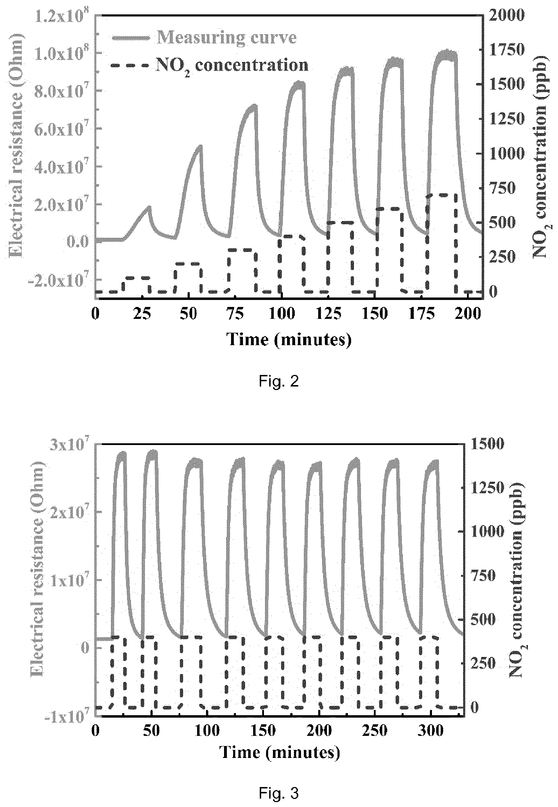

Fig.2 illustrates the sensing response of the sensor towards 100-700 ppb of NO₂ gas. The sensor exhibits an excellent response to ppb-level of NO₂ gas. The detection limit is very low, and the sensor response is about 14.3 even for 100 ppb of NO₂ gas. The response time is about 1.5 min; the recovery time is 3 min. The sensing property of the sensor is comparable to prior art sensors working at high temperatures.

Fig.3 shows a repeatability test of the sensor towards about 400 ppb of NO₂ gas at room temperature. The result demonstrates that the sensor possesses a good repeatability at room temperature. For high temperature gas sensors, humidity is not a problem because water molecules will evaporate at elevated temperatures. In contrast, water vapour may be present on the material surface at room temperature and thus humidity variation might affect the sensing behaviour in this case. Light illumination may be used to eliminate the influence of any water molecules.

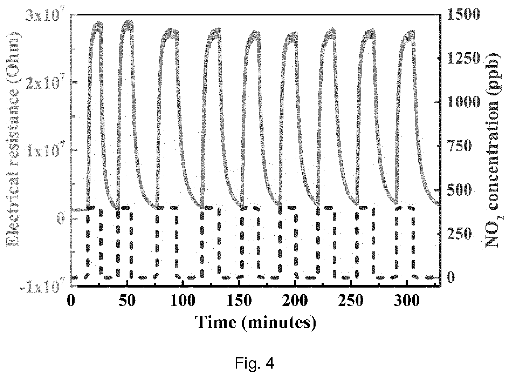

Fig.4 shows the effect of humidity on the sensing performance towards 400 ppb NO₂ in the humidity range of 0-80% at room temperature and shows that the effect of humidity on the sensitivity is insignificant. The base resistance decreases when humidity increases, especially from 0 to 20%. When it further increases, the decrease in base resistance becomes not obvious. As a result, the prepared sensor is substantially insensitive to humidity variations.

## Claims

1. A gas sensor (1) configured to detect a target gas in a gaseous atmosphere, the gas sensor comprising:
- a transparent substrate (12);
- a gas sensitive detection layer supported by the transparent substrate (12), the gas sensitive detection layer comprising i) a gas sensitive detection material (10) having an electrical impedance which is sensitive to the target gas and ii) connections (11) configured to allow for detection of electrical impedance of the gas sensitive detection material;
- a light source (13) configured to provide light to the gas sensitive detection layer through the transparent substrate (12);
the gas sensor (1) being **characterized in that** the gas sensitive detection material (10) comprises nanoplatelets of the gas sensitive detection material, and wherein the nanoplatelets are arranged in the form of hollow microspheres.

2. A gas sensor (1) according to claim 1, wherein the gas sensitive detection material (10) is selected from:
i) a metal oxide; and/or
ii) graphene and/or graphene oxide; and/or
iii) a combination of a metal oxide with an organic material;
and preferably wherein the metal oxide is a doped metal oxide and/or a sub-stoichiometric metal oxide, notably wherein the metal is selected from Zn, Sn, W, and/or Ni.

3. A gas sensor (1) according to any of claims 1 to 2, wherein the transparent substrate (12) is selected from:
i) a flexible substrate, particularly a plastics film, notably a plastics film selected from a PET film, a PE film, a PEN film, a polymer film, a non-crystalline polymer film, a polylactide film; or
ii) a rigid substrate, notably a glass substrate.

4. A gas sensor (1) according to any of claims 1 to 3, wherein the connections (11) configured to allow for detection of electrical impedance of the gas sensitive detection material (10) comprise a pair of spaced electrodes, each of which is electrically connected to the gas sensitive detection material (10).

5. A gas sensor (1) according to any of claims 1 to 4, wherein the connections (11) configured to allow for detection of electrical impedance of the gas sensitive detection material (10) are supported by the transparent substrate (12) and overlaid by the gas sensitive detection material (10).

6. A gas sensor (1) according to any of claims 1 to 5, wherein the target gas to be detected by the gas is selected from NO₂, Os, H₂, SO₂, H₂S, CO and/or VOC gas and/or wherein the gaseous atmosphere is air.

7. A gas sensor (1) according to any of claims 1 to 6, wherein the light source (13) comprises a light-emitting diode.

8. A gas sensor (1) according to any of claims 1 to 7, wherein the light source (13) is a monochromatic light source, preferably a blue light emitting source, more preferably a blue light emitting source configured to provide a blue light in the range 460-490 nm.

9. A gas sensor (1) according to any of claims 1 to 8, wherein the hollow microspheres have an external mean diameter of less than 20 µm.

10. A gas sensor (1) according to claim 9, wherein the nanoplatelets of the gas sensitive detection material (10) have a thickness of less than 0.3 µm, preferably in the range 50 - 100 nm.

11. A gas sensor (1) according to any of claims 1 to 10, wherein the gas sensor further comprises a gas filter between the gas sensitive detection material and the gaseous atmosphere, notably a gas filter configured to prevent or reduce the concentration of one or more selected gasses from the gaseous atmosphere from contacting the gas sensitive detection layer.

12. A gas sensor (1) according to any of claims 1 to 11, wherein the gas sensor is configured to detect
- a concentration of the target gas of less than 10 ppm in the gaseous atmosphere; and/or
- a change of electrical impedance of the gas sensitive detection layer of at least 500 %, preferably at least 5000%, more preferably at least 10000%, in response to a concentration of target gas in the gaseous atmosphere which is less than 1 ppm.

13. A gas sensor (1) according to any of claims 1 to 12, wherein the gas sensor is configured to detect a electrical impedance in the range 1kΩ to 100MΩ.

14. A gas sensor (1) according to any of claims 1 to 13, wherein the gas sensor is configured to detect a target gas in the gaseous atmosphere at 20°C.

15. A method of detecting a target gas in a gaseous atmosphere, notably NO₂ gas in air, the method comprising:
- arranging a gas sensor (1) in accordance with any preceding claim in the gaseous atmosphere;
- providing light to the gas sensitive detection layer through the transparent substrate (12) from the light source (13);
- detecting the presence and/or the concentration of the target gas in gaseous atmosphere by monitoring the electrical impedance of the gas sensitive detection layer.

## Patentansprüche

1. Gassensor (1), der zum Detektieren eines Zielgases in einer gashaltigen Atmosphäre ausgestaltet ist, wobei der Gassensor umfasst:
- ein transparentes Substrat (12);
- eine gassensible Detektionsschicht, die vom transparenten Substrat (12) getragen ist, wobei die gassensible Detektionsschicht umfasst 1) ein gassensibles Detektionsmaterial (10) mit einer elektrischen Impedanz, die sensibel für das Zielgas ist und ii) Anschlüsse (11), die ausgestaltet ist, um die Detektion der elektrischen Impedanz des gassensiblen Detektionsmaterials zu ermöglichen;
- eine Lichtquelle (13), die ausgestaltet ist, um der gassensiblen Detektionsschicht Licht durch das transparente Substrat (12) bereitzustellen;
wobei der Gassensor (1) **dadurch gekennzeichnet ist, dass** das gassensible Detektionsmaterial (10) Nanoplättchen des gassensiblen Detektionsmaterials umfasst und wobei die Nanoplättchen in der Form von hohlen Mikrosphären angeordnet sind.

2. Gassensor (1) gemäß Anspruch 1, wobei das gassensible Detektionsmaterial (10) ausgewählt ist aus:
i) einem Metalloxid; und/oder
ii) Graphen und/oder Graphenoxid; und/oder
iii) einer Kombination eines Metalloxids mit einem organischen Material;
und wobei das Metalloxid bevorzugt ein dotiertes Metalloxid und/oder ein substöchiometrisches Metalloxid ist, wobei insbesondere das Metall ausgewählt ist aus Zn, Sn, W und/oder Ni.

3. Gassensor (1) gemäß irgendeinem der Ansprüche 1 oder 2, wobei das transparente Substrat (12) ausgewählt ist aus:
i) einem flexiblen Substrat, insbesondere einer Plastikfolie, insbesondere einer Plastikfolie, die aus einer PET-Folie, einer PE-Folie, einer PEN-Folie, einer Polymer-Folie, einer nicht kristallinen Polymer-Folie, einer Polyactid-Folie ausgewählt ist; oder
ii) einem steifen Substrat, insbesondere einem Glassubstrat.

4. Gassensor (1) gemäß irgendeinem der Ansprüche 1 bis 3, wobei die Anschlüsse (11), die ausgestaltet sind, um die Detektion der elektrischen Impedanz des gassensiblen Detektionsmaterials (10) zu ermöglichen, ein Paar beabstandeter Elektroden umfassen, von denen jede elektrisch an das gassensible Detektionsmaterial (10) angeschlossen ist.

5. Gassensor (1) gemäß irgendeinem der Ansprüche 1 bis 4, wobei die Anschlüsse (11), die ausgestaltet sind, um die Detektion der elektrischen Impedanz des gassensiblen Detektionsmaterials (10) zu ermöglichen, von dem transparenten Substrat (12) getragen sind und mit dem gassensiblen Detektionsmaterial (10) überlagert sind.

6. Gassensor (10) gemäß irgendeinem der Ansprüche 1 bis 5, wobei das Zielgas, das von dem Gas zu detektieren ist, ausgewählt ist aus NO₂, O₃, H₂, SO₂, H₂S, CO und/oder VOC-Gas und/oder wobei die gashaltige Atmosphäre Luft ist.

7. Gassensor (1) gemäß irgendeinem der Ansprüche 1 bis 6, wobei die Lichtquelle (13) eine Lichtemissionsdiode umfasst.

8. Gassensor (1) gemäß irgendeinem der Ansprüche 1 bis 7, wobei die Lichtquelle (13) eine monochromatische Lichtquelle, bevorzugt eine blaue Lichtemissionsquelle, weiter bevorzugt eine blaue Lichtemissionsquelle ist, die ausgestaltet ist, um ein blaues Licht im Bereich von 460 - 490 nm bereitzustellen.

9. Gassensor (1) gemäß irgendeinem der Ansprüche 1 bis 8, wobei die hohlen Mikrosphären einen durchschnittlichen Außendurchmesser von weniger als 20 µm aufweisen.

10. Gassensor (1) gemäß Anspruch 9, wobei die Nanoplättchen des gassensiblen Detektionsmaterials (10) eine Dicke von weniger als 0,3 µm, bevorzugt im Bereich von 50 - 100 nm, aufweisen.

11. Gassensor (1) gemäß irgendeinem der Ansprüche 1 bis 10, wobei der Gassensor weiterhin zwischen dem gassensiblen Detektionsmaterial und der gashaltigen Atmosphäre einen Gasfilter, insbesondere einen Gasfilter umfasst, der ausgestaltet ist, um die Konzentration von einem oder mehreren ausgewählten Gas(en) aus der gashaltigen Atmosphäre daran zu hindern, die gassensible Detektionsschicht zu kontaktieren oder dies zu reduzieren.

12. Gassensor (1) gemäß irgendeinem der Ansprüche 1 bis 11, wobei der Gassensor ausgestaltet ist zum Detektieren
- einer Konzentration des Zielgases von weniger als 10 ppm in der gashaltigen Atmosphäre; und/oder
- einer Änderung der elektrischen Impedanz der gassensiblen Detektionsschicht von wenigstens 500 %, bevorzugt wenigstens 5.000 %, weiter bevorzugt wenigstens 10.000 %, als Antwort auf eine Konzentration von Zielgas in der gashaltigen Atmosphäre, die geringer ist als 1 ppm.

13. Gassensor (1) gemäß irgendeinem der Ansprüche 1 bis 12, wobei der Gassensor ausgestaltet ist, um eine elektrische Impedanz im Bereich von 1 kΩ bis 100 MΩ zu detektieren.

14. Gassensor (1) gemäß irgendeinem der Ansprüche 1 bis 13, wobei der Gassensor ausgestaltet ist, um ein Zielgas in der gashaltigen Atmosphäre bei 20 °C zu detektieren.

15. Verfahren zum Detektieren eines Zielgases in einer gashaltigen Atmosphäre, insbesondere NO₂-Gas in der Luft, wobei das Verfahren umfasst:
- Anordnen eines Gassensors (1) gemäß irgendeinem der voranstehenden Ansprüche in der gashaltigen Atmosphäre;
- Bereitstellen von Licht aus der Lichtquelle (13) für die gassensible Detektionsschicht durch das transparente Substrat (12) ;
- Detektieren des Vorhandenseins und/oder der Konzentration des Zielgases in der gashaltigen Atmosphäre durch Überwachen der elektrischen Impedanz der gassensiblen Detektionsschicht.

## Revendications

1. Capteur de gaz (1) configuré de manière à détecter un gaz cible dans une atmosphère gazeuse, le capteur de gaz comprenant :
- un substrat transparent (12) ;
- une couche de détection sensible au gaz qui est supportée par le substrat transparent (12), la couche de détection sensible au gaz comprenant : i) un matériau de détection sensible au gaz (10) qui présente une impédance électrique sensible au gaz cible ; et ii) des connexions (11) configurées de manière à permettre la détection de l'impédance électrique du matériau de détection sensible au gaz ; et
- une source de lumière (13) qui est configuré de manière à appliquer de la lumière à la couche de détection sensible au gaz à travers le substrat transparent (12),
le capteur de gaz (1) étant **caractérisé en ce que** le matériau de détection sensible au gaz (10) comprend des nanoplaquettes du matériau de détection sensible au gaz, et dans lequel les nanoplaquettes sont agencées sous la forme de microsphères creuses.

2. Capteur de gaz (1) selon la revendication 1, dans lequel le matériau de détection sensible au gaz (10) est sélectionné parmi :
i) un oxyde de métal ; et/ou
ii) le graphène et/ou l'oxyde de graphène ; et/ou
iii) une combinaison d'un oxyde de métal avec un matériau organique,
et de préférence dans lequel l'oxyde de métal est un oxyde de métal dopé et/ou un oxyde de métal sous-stœchiométrique, en particulier dans lequel le métal est sélectionné parmi le Zn, le Sn, le W et/ou le Ni.

3. Capteur de gaz (1) selon l'une quelconque des revendications 1 à 2, dans lequel le substrat transparent (12) est sélectionné parmi :
i) un substrat souple, en particulier un film plastique, en particulier un film plastique sélectionné parmi un film de PET, un film de PE, un film de PEN, un film polymère, un film polymère non cristallin et un film de polylactide ; ou
ii) un substrat rigide, en particulier un substrat de verre.

4. Capteur de gaz (1) selon l'une quelconque des revendications 1 à 3, dans lequel les connexions (11) configurées de manière à permettre la détection de l'impédance électrique du matériau de détection sensible au gaz (10) comprennent une paire d'électrodes espacées, dans lequel chacune de celles-ci est connectée électriquement au matériau de détection sensible au gaz (10) .

5. Capteur de gaz (1) selon l'une quelconque des revendications 1 à 4, dans lequel les connexions (11) configurées de manière à permettre la détection de l'impédance électrique du matériau de détection sensible au gaz (10) sont supportées par le substrat transparent (12) et sont recouvertes par le matériau de détection sensible au gaz (10).

6. Capteur de gaz (1) selon l'une quelconque des revendications 1 à 5, dans lequel le gaz cible à détecter par le gaz est sélectionné parmi les gaz NO₂, O₃, H₂, SO₂, H₂S, CO et/ou COV et/ou dans lequel l'atmosphère gazeuse est l'air.

7. Capteur de gaz (1) selon l'une quelconque des revendications 1 à 6, dans lequel la source de lumière (13) comprend une diode électroluminescente.

8. Capteur de gaz (1) selon l'une quelconque des revendications 1 à 7, dans lequel la source de lumière (13) est une source de lumière monochromatique, de préférence une source émettrice de lumière bleue, de préférence encore une source émettrice de lumière bleue qui est configuré de manière à produire une lumière bleue comprise dans la gamme 460 nm à 490 nm.

9. Capteur de gaz (1) selon l'une quelconque des revendications 1 à 8, dans lequel les microsphères creuses présentent un diamètre moyen extérieur qui est inférieur à 20 µm.

10. Capteur de gaz (1) selon la revendication 9, dans lequel les nanoplaquettes du matériau de détection sensible au gaz (10) présentent une épaisseur qui est inférieure à 0,3 um, et qui est de préférence comprise entre 50 nm et 100 nm.

11. Capteur de gaz (1) selon l'une quelconque des revendications 1 à 10, dans lequel le capteur de gaz comprend en outre un filtre à gaz entre le matériau de détection sensible au gaz et l'atmosphère gazeuse, en particulier un filtre à gaz qui est configuré de manière à réduire ou à empêcher la concentration d'un ou de plusieurs gaz sélectionné(s) de l'atmosphère gazeuse d'entrer en contact avec la couche de détection sensible au gaz.

12. Capteur de gaz (1) selon l'une quelconque des revendications 1 à 11, dans lequel le capteur de gaz est configuré de manière à détecter :
- une concentration en gaz cible inférieure à 10 ppm dans l'atmosphère gazeuse ; et/ou
- un changement d'impédance électrique de la couche de détection sensible au gaz d'au moins 500 %, de préférence d'au moins 5000 %, de préférence encore d'au moins 10 000 %, en réponse à une concentration en gaz cible dans l'atmosphère gazeuse qui est inférieure à 1 ppm.

13. Capteur de gaz (1) selon l'une quelconque des revendications 1 à 12, dans lequel le capteur de gaz est configuré de manière à détecter une impédance électrique qui est comprise entre 1 kΩ et 100 MΩ.

14. Capteur de gaz (1) selon l'une quelconque des revendications 1 à 13, dans lequel le capteur de gaz est configuré de manière à détecter un gaz cible dans l'atmosphère gazeuse à 20°C.

15. Procédé de détection d'un gaz cible dans une atmosphère gazeuse, en particulier de gaz NO₂ dans l'air, le procédé comprenant les étapes suivantes :
- agencer un capteur de gaz (1) selon l'une quelconque des revendications précédentes dans l'atmosphère gazeuse ;
- appliquer de la lumière à la couche de détection sensible au gaz à travers le substrat transparent (12) à partir de la source de lumière (13) ; et
- détecter la présence et/ou la concentration en gaz cible dans l'atmosphère gazeuse en surveillant l'impédance électrique de la couche de détection sensible au gaz.
